(19) Europäisches Patentamt
European Patent Office
Office européen des brevets

(11) EP 4 081 576 B1

(12) # EUROPEAN PATENT SPECIFICATION

(45) Date of publication and mention of the grant of the patent:
**06.11.2024 Bulletin 2024/45**

(21) Application number: **19957065.6**

(22) Date of filing: **23.12.2019**

(51) International Patent Classification (IPC):
***C08J 3/075*** (2006.01) ***C08L 5/08*** (2006.01)
***A61L 27/20*** (2006.01)

(52) Cooperative Patent Classification (CPC):
(C-Sets available)
**C08B 37/0072; A61L 27/26; C08B 37/0021; C08J 3/075; C08L 5/02; C08L 5/08;** A61L 2400/06; A61L 2430/34; A61L 2430/40; C08J 2305/02; C08J 2305/08 (Cont.)

(86) International application number:
**PCT/CN2019/127320**

(87) International publication number:
**WO 2021/127807 (01.07.2021 Gazette 2021/26)**

(54) **DUAL-CROSSLINKED HYDROGEL AND PREPARATION METHOD THEREOF**

DOPPELT VERNETZTES HYDROGEL UND VERFAHREN ZU SEINER HERSTELLUNG

HYDROGEL À DOUBLE RÉTICULATION ET SON PROCÉDÉ DE PRÉPARATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**02.11.2022 Bulletin 2022/44**

(73) Proprietor: **Evonik Operations GmbH**
**45128 Essen (DE)**

(72) Inventors:
- **FU, Jun**
  **Guangzhou 510275 (CN)**
- **ZHANG, Hua**
  **Ningbo, Zhejiang (CN)**
- **JIANG, Jinhua**
  **Shanghai 200233 (CN)**
- **FENG, Jing**
  **Shanghai 201199 (CN)**

(74) Representative: **Evonik Patent Association**
**c/o Evonik Industries AG**
**IP Management**
**Bau 1042A/PB 15**
**Paul-Baumann-Straße 1**
**45772 Marl (DE)**

(56) References cited:
**WO-A1-2019/210496 CN-A- 101 296 709**
**CN-A- 104 910 392 CN-A- 104 910 396**
**CN-A- 110 240 712 US-A1- 2017 065 746**
**US-A1- 2019 365 902**

- **WALIMBE TANAYA ET AL: "A Review of Hyaluronic Acid and Hyaluronic Acid-based Hydrogels for Vocal Fold Tissue Engineering", JOURNAL OF VOICE, ELSEVIER SCIENCE, US, vol. 31, no. 4, 2 March 2017 (2017-03-02), pages 416 - 423, XP085116145, ISSN: 0892-1997, DOI: 10.1016/J.JVOICE.2016.11.014**
- **LUO YING ET AL: "Injectable hyaluronic acid-dextran hydrogels and effects of implantation in ferret vocal fold", JOURNAL OF BIOMEDICAL MATERIALS RESEARCH PART B: APPLIED BIOMATERIALS, vol. 93B, no. 2, 11 February 2010 (2010-02-11), US, pages 386 - 393, XP093064391, ISSN: 1552-4973, DOI: 10.1002/jbm.b.31593**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 081 576 B1

- **CHEN JIAQING ET AL: "Modified hyaluronic acid hydrogels with chemical groups that facilitate adhesion to host tissues enhance cartilage regeneration", BIOACTIVE MATERIALS, vol. 6, no. 6, 1 June 2021 (2021-06-01), pages 1689 - 1698, XP093064405, ISSN: 2452-199X, DOI: 10.1016/j.bioactmat.2020.11.020**

(52) Cooperative Patent Classification (CPC): (Cont.)

C-Sets
**A61L 27/26, C08L 5/08**

## Description

## Technical Field

The invention relates to an injectable hydrogel with secondary autonomous covalent crosslinking and a preparation method thereof.

## Background art

Glycosaminoglycans (GAGs) are long linear (unbranched) polysaccharides consisting of repeating disaccharide (double sugar) units. Glycosaminoglycans have good biocompatibility. Hyaluronic acid (HA; conjugate base hyaluronate), also called hyaluronan, is an anionic, nonsulfated glycosaminoglycan. It is a linear polysaccharide that consists of alternating units of a repeating disaccharide, β-1,4-D-glucuronic acid-β-1,3-N -acetyl-D-glucosamine. It is abundant in cartilage and skin and plays a key structural role in the organization of the extracellular matrix as an organizing structure for the assembly of a proteoglycan. Viscous solutions of high molecular-weight HA and its derivatives are being used in therapy for promoting wound healing in various tissues, such as a surgical aid in eye and skin. However, these solution systems are limited in application, due to undesirable loss of material from the injection site and minimal control over important material properties (e.g., mechanics and degradation).

Injectable hydrogels may be surgically implanted to fit variable target sites in patients using minimally invasive methods, making them particularly attractive for clinical use. For these purposes, a variety of injectable HA-based hydrogels have been explored by numerous chemical crosslinking mechanisms including photo-crosslinking, dynamic-chemistry, physical-assembly and non-covalent interactions.

For example, in CN104892962A, HA was functionalized with thiol groups and subjected to a one-step crosslinking protocol for an injectable HA hydrogel basing on oxidation-reduction reactions between thiols and disulfide bond. Unfortunately, these materials are inherently limited in that they typically exhibit low mechanical strength and may exhibit rapid degradation.

Ying Luo et.al. (Journal of Biomedical Materials Research B: Applied Biomaterials | May 2010 Vol 93b, Issue 2 387) disclosed an injectable hyaluronic acid-dextran hydrogel, wherein hyaluronic acid (HA) and dextran were chemically modified and subsequently crosslinked via formation of hydrazone (acylhydrazone) bonds in phosphate buffer. The compressive moduli of the hydrogels were around or above 10 kPa.

Hydrogels formed through covalent means display great versatility with the allowed inclusion of controlled network degradation and mechanical strength. In CN106188584A, a UV-crosslinked, stiff HA hydrogel was prepared by free-radical photopolymerization of methacrylates, where HA were functionalized with both 2-aminoethyl methacrylate hydrochloride and L-lysine. However, as a result of the single covalent bonding structure, hydrogels are unable of self-healing, which limits them in their applications as an injectable material.

Hoang D. Lu et.al. (Adv Healthc Mater. 2013 Jul; 2(7):1028-36) disclosed a secondary photo-crosslinking of injectable shear-thinning dock-and-lock hydrogel, wherein the hydrogel was stabilized through light-initiated radical polymerization of methacrylate functional groups to tune gel mechanics and erosion kinetics. By tuning material compositions, hydrogels with storage modulus (G') as high as ~ 1 kPa could be formed.

For more physically demanding applications, non-covalently and covalently crosslinked systems may be more appropriate to modulate the injectability, self-healing properties and the degradation and mechanical strength. For example, in CN104910396A, an injectable dual-crosslinked hydrogel system was prepared from amino-/vinyl- HA and oxidized HA. The hydrogels first undergo dynamic Schiff-based crosslinking through amino- and aldehyde- groups, then a secondary covalent crosslinking occurs in situ via vinyl- to stabilize the network. The compression moduli were around 7.4kPa~18.9kPa.

Sudhir Khetan et.al. (Nat Mater. 2013 May;12(5):458-65) disclosed a MeHA hydrogel with compression moduli around 4~91 kPa. The final storage modulus would be even lower according to the relationship between compression modulus and storage modulus: $E = 2 \times (1+\mu)\ G$. Here, $E$ is Young's modulus. $\mu$ is Poisson's ratio, which is from 0 to 0.5. G is shear modulus of elasticity.

In particular, a disadvantage with the Schiff-based reaction is exceedingly fast gelation, which may result in premature crosslinking and delivery failure. For ease of clinical application, the hydrogel must therefore undergo crosslinking with gentle reaction kinetics to prevent premature crosslinking and delivery failure.

There was thus the problem of providing a novel, mild HA-dextran hydrogel system which can self-heal immediately after shear induced flow, is cytocompatible, and can be stabilized through light-initiated radical polymerization of methacrylate functional groups to tune gel mechanics and degradation kinetics. Further, there was the problem of providing a process for the production of such hydrogels.

Stiffness has been regarded as a key metric for how the matrix resists cellular traction forces to regulate stem cell fate. For example, stiff substrates with moduli in the range of 30-35 kPa promote stem cell differentiation into

osteoblasts. On the other hand, the phenotype of stem cell can be better maintained when they are cultured on soft substrates with a modulus of 0.5 kPa, as compared to those with a modulus of 30 kPa. From soft fat to stiff articular cartilage and bone, the moduli of these tissue are from 0.02 kPa to 950 kPa (see, Akon Higuchi et al. Chem. Rev. 2013, 113, 3297-3328; Akon Higuchi et al. Chem. Rev. 2012, 112, 4507-4540; and Biji Balakrishnan et al. Chem. Rev. 2011, 111, 4453-4474). Therefore, a key goal is to achieve high moduli for hydrogel produced from natural materials thus with good biocompatibility and good tissue regeneration.

**Summary of the invention**

To address these inherent limitations of current injectable hydrogel systems, a novel, mild hydrogel system was developed by using mild dual-crosslinking conditions. To accomplish this, according to the present invention, a glycosaminoglycan such as HA is first functionalized with methacrylate and hydrazide groups, and a second-polysaccharide such as dextran is oxidized to obtain aldehyde groups. The mixture of functionalized glycosaminoglycan and second-polysaccharide-aldehyde first undergo gentle dynamic covalent acylhydrazone bond crosslinking through hydrazide and aldehyde groups to obtain a dynamic acylhydrazone bond cross-linked hydrogel. Acylhydrazone bond formation is an efficient, biocompatible chemistry, often used for bioconjugation, but also can be tuned to be dynamically covalent, depending on the chemical structures. See C. Sun et al. Polymer 160 (2019) 246-253 for more description on acylhydrazone bond. This mechanism permits them to possess desirable properties like shear-thinning and rapidly self-healing. After injection of the dynamic acylhydrazone bond cross-linked hydrogel into a target site, a secondary covalent crosslinking occurs in situ via photopolymerization of methacrylates to stabilize the network and modulate mechanics to obtain the dual-crosslinked hydrogel.

The properties of the dual-crosslinked hydrogels permit them to be used as injectable and photo-stabilizing cell carriers. Cells such as stem cells can be homogenously encapsulated within the hydrogels under constant conditions by, for example, simply resuspending cells with for example one glycosaminoglycan component dissolved in growth media and subsequently mixing with the second polysaccharide component such as dextran-aldehyde. The dual-crosslinked hydrogels have good cytocompatibility and viable cells may be remained above 90 % for 14 days.

Without wishing to be bound by theory, it is believed that the dual-crosslinking, i.e., acylhydrazone crosslinking and methacrylate covalent crosslinking makes the dual-crosslinked hydrogel of the invention possesses advantages such as improved mechanical properties. The dual-crosslinked hydrogel of the invention may have a final storage modulus up to 1000 kPa or more. Apparently, there is a synergetic effect between acylhydrazone crosslinking and methacrylate covalent crosslinking as none of the acylhydrazone crosslinking and methacrylate covalent crosslinking alone may achieve such high modulus. Such high modulus is particularly important and valuable for the applications such as scaffold for cartilage in tissue engineering. Thus, the dual-crosslinked hydrogel of the invention may be applied in applications which requires a high final storage modulus.

Compared with conventional hydrogels with dynamic acylhydrazone-crosslinking only, the dual-crosslinked hydrogel systems exhibit a slow reduction in mass loss at all observed time points. Moreover, the final storage modulus from photopolymerization of the dual-crosslinked hydrogel significantly increases from 0.1 kPa to 1000 kPa or more, which is very surprising for and beyond expectation of a person skilled in the art.

The invention provides a process to prepare a dual-crosslinked hydrogel, comprising the following steps,

1) functionalizing a glycosaminoglycan such as hyaluronic acid with a methacrylate on hydroxyl group and with an dihydrazide on carboxyl group to obtain a methacrylate and hydrazide group functionalized glycosaminoglycan, which can be either a glycosaminoglycan with both methacrylate and hydrazide group modification, or a glycosaminoglycan with methacrylate modification only and a glycosaminoglycan with hydrazide group modification only, wherein the degree of methacrylation is 5-200%, preferably 85-165%, and the degree of hydrazide group modification is 8-70%, preferably 10-65%, more preferably 10-40%; oxidizing a second-polysaccharide such as dextran to obtain a second-polysaccharide with aldehyde groups, the degree of aldehyde modification in the second-polysaccharide is 10-95%, preferably 85-95%;

2) crosslinking the methacrylate and hydrazide group functionalized glycosaminoglycan and the second-polysaccharide-aldehyde in an aqueous solvent, such as phosphate buffer saline (PBS) or a cell culture medium, to form a dynamic acylhydrazone bond cross-linked hydrogel; and

3) photopolymerizing the dynamic acylhydrazone bond cross-linked hydrogel by irradiation under the presence of a photoinitiator.

The dual-crosslinked hydrogel of the invention comprises a first dynamic acylhydrazone bond crosslinking between hydrazide groups of hydrazide groups modified glycosaminoglycan and aldehyde groups of a second-polysaccharide-aldehyde, and a secondary covalent crosslinking formed in situ via photopolymerization of the methacrylate groups modified on the glycosaminoglycan.

The glycosaminoglycan of the invention is water soluble. The glycosaminoglycan is selected from glycosaminoglycans that may be modified by with a methacrylate on a hydroxyl group and with a dihydrazide on a carboxyl group. The glycosaminoglycan is preferably selected from the group consisting of hyaluronic acid, chondroitin sulfate (e.g. chondroitin-6-sulfate, and chondroitin-4-sulfate), dermatan sulfate, heparan sulfate and heparin, especially hyaluronic acid. Unless otherwise specified, the term "hyaluronic acid" in the invention refers to hyaluronic acid or a salt thereof, such as sodium hyaluronate. The salt of hyaluronic acid refers to water-soluble salt of hyaluronic acid. Non-limiting examples may be selected from sodium hyaluronate, hyaluronic acid potassium salt, tetrabutylammonium hyaluronate (HA-TBA), etc.

The second-polysaccharide of the invention is a water-soluble polysaccharide. The second-polysaccharide comprises a 1,4-linkage and/or a 1,6-linkage. In addition, the second-polysaccharide comprises an ortho-hydroxyl on C2, C3 or C4 of the sugar ring of the polysaccharide. The second-polysaccharide does not include those with 1,3-linkage only. The second-polysaccharide may be glucans such as β-1,3/1,6-glucan, α-1,6-glucan with α-1,3-branches, α-1,6-glucan, α -1,4/1,6-glucan, alginate and pectin, etc. Non-limiting examples of the glucan include dextran, laminarin (β-1,3- and β-1,6-glucan), water soluble cellulose derivatives, especially dextran.

The term "methacrylate" refers to hydrolyzable methacrylates. Non-limiting examples of methacrylate include methacrylic anhydride, 2-aminoethyl methacrylate hydrochloride, glycidyl methacrylate.

The dihydrazide in the invention refers to a dihydrazide that may, after hydrazide group modification of the glycosaminoglycan, further react with an aldehyde group to form an acylhydrazone bond. The dihydrazide may be selected from water-soluble dihydrazides. Non-limiting examples may be selected from adipic dihydrazide, ethanedihydrazide, oxalyldihydrazide, dodecanedioic dihydrazide.

In some embodiments, the dual-crosslinked hydrogel is a dual-crosslinked hyaluronic acid-dextran hydrogel.

Methacrylate may react with the free hydroxyl groups at different positions such as C6 of the hyaluronic acid repeating unit, although hydroxyl group of C6 is typically the most reactive one.

In the invention, the term "second-polysaccharide with aldehyde groups" is used interchangeably with "second-polysaccharide-aldehyde". Both terms mean second-polysaccharide with aldehyde groups oxidized from hydroxyl groups on the original second-polysaccharide molecule. Similarly, the term "dextran with aldehyde groups" is used interchangeably with "dextran-aldehyde". Both terms mean dextran with aldehyde groups oxidized from hydroxyl groups on the original dextran molecule.

The term "degree of methacrylation" refers to:

(the mole of methacrylated hydroxyl groups / the total mole number of repeating units contained in the polymer) * 100%

See S.K. Seidlits et al. Biomaterials 31 (2010) 3930-3940 for more description on methcarylation degree and the determination method thereof.

The degree of methacrylation may be 5-200%, preferably 85-165%, for example 100-165%, 110-165%,120-165%,130-165%, 140-165%, or 150-165%.

The term "degree of hydrazide group modification" refers to:

(the mole of hydrazide group modified repeating units/ the mole of repeating units in the polymer) * 100%.

See Paul Bulpitt et al. Journal of biomedical materials research, 47(2), 152-169 for the determination method thereof.

The degree of hydrazide group modification may be 8-70%, preferably 10-65%, for example 10-40%, 20-65%, or 20-40%.

When adipic dihydrazide (ADH) and dextran is used, acylhydrazone bonds are formed between the aldehyde groups of dextran-aldehyde (oxidized dextran or ODEX) and the acylhydrazide bonds of ADH in step 2).

A person skilled in the art may control the oxidation degree of the second-polysaccharide-aldehyde, e.g. through reaction time and the amount of the oxidant. The degree of aldehyde modification in the second-polysaccharide such as dextran product may be determined by e.g. measuring the number of aldehyde groups in the polymer using t-butyl carbazate and trinitrobenzenesulfonic acid, see for example, Jia X, Colombo G, Padera R, Langer R, Kohane DS. Prolongation of sciatic nerve blockade by in situ cross-linked hyaluronic acid. Biomaterials 2004; 25:4797-4804; or Bouhadir KH, Housman DS, Mooney DJP. Synthesis of crosslinked poly(aldehyde guluronate) hydrogels. Polymer 1999; 40: 3575-3584.

The degree of aldehyde modification in the second-polysaccharide maybe 10-95%, for example 50-95%, 60-95%, 70-95%, or 80-95%, preferably 85-95%.

In order to obtain optimized gelation, a person skilled in the art may adjust the concentration of the modified glycosaminoglycan and modified second-polysaccharide; preferably the concentration of modified glycosaminoglycan such as modified hyaluronic acid is 0.5-3wt.%, preferably 1~2wt.%, the concentration of second-polysaccharide-aldehyde such as dextran-aldehyde is 4-8wt.%, preferably 4~6wt.% based on the total weight of the reaction mixture in step 2).

In some embodiments, the glycosaminoglycan, such as hyaluronic acid may have a molecular weight of 50-2000

kDa, preferably 50-1000 kDa.

In some embodiments, the second-polysaccharide, such as dextran may have a molecular weight of 10-1000 kDa, such as 10-100 kDa,10~70 kDa, preferably 10-20 kDa.

In step 1), glycosaminoglycan such as hyaluronic acid is modified with methacrylate to obtain a methacrylated glycosaminoglycan, then further modified with hydrazide groups. Alternatively, glycosaminoglycan may be modified with methacrylate and hydrazide groups separately (i.e., not in the same molecule), then in step 2) crosslinking both the methacrylated glycosaminoglycan and hydrazide modified glycosaminoglycan with the second-polysaccharide-aldehyde to form a dynamic acylhydrazone bond cross-linked hydrogel. In addition, in step 1), the second-polysaccharide is oxidized to prepare a second-polysaccharide-aldehyde.

In step 2), the modified glycosaminoglycan and second-polysaccharide -aldehyde is crosslinked via formation of acylhydrozone bonds, preferably in a phosphate buffer saline (PBS) or a cell culture medium.

In step 3), the methacrylate functional groups of the dynamic acylhydrazone bond cross-linked hydrogel are polymerized. In some examples, the polymerization is a light-initiated radical polymerization.

The irradiation may be 320-500 nm irradiation, sunlight irradiation, etc., preferably 405 nm blue light. In some embodiments, the dynamic acylhydrazone bond cross-linked hydrogels were crosslinked by using 405 nm blue-light, at a power of 2-4 W, a light intensity of 6~10 $mW/cm^2$ for 1-3 min.

The photoinitiator may be those conventional in the art, for example lithium phenyl-2, 4, 6-trimethylbenzoylphosphinate (LAP). In some embodiments, the photoinitiator may be dissolved in the aqueous solvent of step 2).

The invention further provides a dual-crosslinked hydrogel prepared according to the above process of the invention.

The invention further provides a hydrogel precursor to prepare a dual-crosslinked hydrogel, wherein the hydrogel precursor is prepared by a method comprising the following steps:

1) functionalizing a glycosaminoglycan such as hyaluronic acid with methacrylate on hydroxyl group and with a hydrazide on carboxyl group to obtain a methacrylate and hydrazide group functionalized glycosaminoglycan, which can be either a glycosaminoglycan with both methacrylate and hydrazide group modification, or a glycosaminoglycan with methacrylate modification only and a glycosaminoglycan with hydrazide group modification only, wherein the degree of methacrylation is 5-200%, preferably 85-165%, and the degree of hydrazide group modification is 8-70%, preferably 10-65%, more preferably 10-40%; oxidizing a second-polysaccharide such as dextran to obtain a second-polysaccharide with aldehyde groups, the degree of aldehyde modification in the second-polysaccharide is 10-95%, preferably 85-95%; and

2) crosslinking the methacrylate and hydrazide group functionalized glycosaminoglycan and the second-polysaccharide with aldehyde groups in an aqueous solvent, such as phosphate buffer saline (PBS) or a cell culture medium, to form a dynamic acylhydrazone bond cross-linked hydrogel.

The hydrogel precursor is the dynamic acylhydrazone bond cross-linked hydrogel prepared in step 2). It is injectable and may be used to prepare the dual-crosslinked hydrogel of the invention.

In use, the hydrogel precursor may optionally carry active ingredients like cells and be injected into the desired part of a subject. Then the hydrogel precursor may be photopolymerized by exposure to irradiation (e.g. 405 nm blue light) in situ, thereby a dual-crosslinked hydrogel may be prepared.

The invention further provides use of the dual-crosslinked hydrogel of the invention or prepared according to the process of the invention or the hydrogel precursor of the invention in tissue engineering, bioapplications such as drug delivery system, biosensors and soft tissue filling agents, etc., or as a carrier of at least one active ingredient such as cell. The cell includes stem cells. The cell carrier may have a high cell viability. Notably, stem cells can be homogenously encapsulated within the dual-crosslinked hydrogel and the viable cells may be remained above 90 % for 14 days. In use, the cells may be incorporated into the dual-crosslinked hydrogel by various ways. For example, the cells may be incorporated into the dual-crosslinked hydrogel by impregnating the dynamic acylhydrazone bond cross-linked hydrogel in a cell suspension; or preferably cells may be added into the reaction mixture before forming the dynamic acylhydrazone bond cross-linked hydrogel, for example cells may be mixed with the functionalized glycosaminoglycan and/or second-polysaccharide with aldehyde groups.

The dual-crosslinked hydrogel with cell may be used as tissue induced scaffold. The dual-crosslinked hydrogel without cell may be used as tissue engineering scaffold.

Chemical crosslinking can be tuned by the dynamic acylhydrazone crosslinking and secondary photo-irradiation, and the extent of chemical crosslinking can be tuned by adjusting irradiation exposure time. The gelation time and equivalent time can be controlled within 20 s-60 s and 10-30 mins, respectively for the dynamic acylhydrazone bond cross-linked hydrogel. With secondary crosslinking, the moduli of the hydrogels can be controlled within 0.1 kPa~2000 kPa, or be controlled from 100 kPa, 200 kPa, 300 kPa, 400 kPa, 500 kPa, 600 kPa, 700 kPa, or 800 kPa, to 1000 kPa,

even up to 1100 kPa, 1200 kPa, 1500 kPa, 1600 kPa, 1700 kPa, 1800 kPa, 1900 kPa, 1950 kPa, or 2000 kPa, by e.g. controlling the degree of degree of methacrylation, the degree of hydrazide group modification, the degree of aldehyde modification, the molecular weight of glycosaminoglycan and second-polysaccharide, the concentration of modified glycosaminoglycan such as hyaluronic acid, the concentration of second-polysaccharide-aldehyde, and the irradiation exposure time between for example 10 s, 20 s, 30 s, 40 s, 50 s, or 60 s to 2 mins, 3 mins, 5 mins, 10 mins, or 20 mins, such as 20 s to 20 mins, 30 s to 20 mins, 40 s to 20 mins, 50 s to 20 mins, 60 s to 20 mins.

In some embodiments, the chemical structure of dual-functionalized HA with dihydrazide- and methacrylate-modification may be schematically shown as formula I:

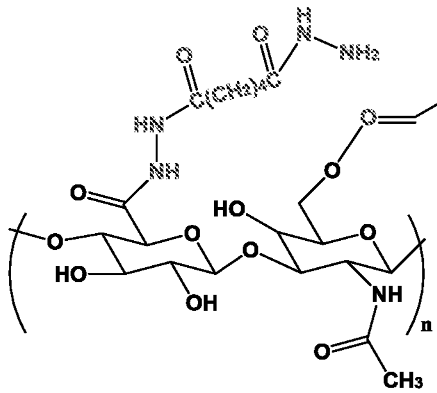

(I)

In some embodiments, the molecular weight of sodium hyaluronate is 50~2000 kDa, for example, 50~1500 kDa, preferably 200-1000 KDa. In one embodiment, the molecular weight of sodium hyaluronate is 200-500 KDa.

In some embodiments, the structure of dextran-aldehyde (oxidized dextran or ODEX) may be schematically shown as formula II:

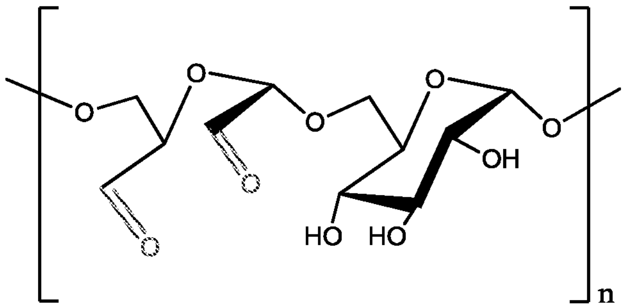

(II)

In some embodiments, the dual-crosslinked hydrogel according to the invention is prepared according to the method as follows,

Preparation of Methacrylated HA (MeHA)

Dissolving sodium hyaluronate (e.g.200-500 kDa) at 0.5-1.5 wt.% in deionized water (DI $H_2O$);
Adding methacrylic anhydride dropwise (~1.5-3 mL per g HA) with stirring at 2~5 °C; Maintaining the stirring mixture at pH 7.5-8.5 by continuously adding 5-10 mM NaOH for 8-12 h;
Reacting overnight at 2~5 °C;
Adding MA (1-2 mL MA per g HA) with pH maintenance for 4-6 h; and
Purifying the methacrylated HA.

In some embodiments, the purification is done bydialyzing the macromer solution against DI $H_2O$ ($M_W$ cutoff 8,000-14,000) for 3-5 days.

The purified methacrylated HA may be frozen at -60 to -80 °C, lyophilized, and stored at -80 °C in powder form.

Preparation of Dihydrazide-HA (DHA-HA) or Dihydrazide-MeHA (DHA-MeHA) Dissolving sodium hyaluronate

or MeHA in $H_2O$ at a concentration of 2-3 mg/mL; Adding a 30-fold molar excess of adipic dihydrazide to this solution;

Adjusting the pH of the reaction mixture to 6.5-7.0, e.g. with 0.1-0.5 M NaOH/0.1-0.5M HCl;
Dissolving 1-Ethyl-3-[3 (dimethylamino)propyl]-carbodiimide (EDC) (1-2 mmol) and 1-hydroxybenzotriazole (HOBt) (1-2 mmol) in dimethylsulfoxide (DMSO)/$H_2O$ (1:1, 1 mL);
After mixing, maintaining the pH of the reaction at 6.5-7.0 e.g. by the addition of 0.1-0.5 M NaOH and allowing the reaction to proceed 12-15 h;
Adjusting the pH to 7.0-7.4, e.g. with 0.1-0.5M NaOH; and
Purifying the obtained derivatized HA.

In some embodiments, the purification is done by exhaustively dialyzing (MW cutoff 8,000-14,000) against distilled $H_2O$ for 3-5 days.
The yield of product may typically be around 80%.

Preparation of dextran-aldehyde

Dissolving dextran in water at a concentration of 200-250 mg/mL;
Adding dropwise an aqueous solution of sodium periodate (0.1-0.5 g dissolved in 5 mL water);
Stirring the obtained mixture for 2 h at room temperature in the dark;
Adding ethylene glycol to inactivate any unreacted periodate; and
Purifying the obtained dextran-aldehyde.

In some embodiments, the purification is done by exhaustive dialysis against water for 3-5 days. Dry product of the dextran-aldehyde may be obtained by freeze drying.
Dual-crosslinked MeHA/DHA-HA/ODEX or DHA-MeHA/ODEX hydrogel formation Preparing MeHA/DHA-HA or DHA-MeHA solutions (ranging from 1.0-2 wt.%) in PBS (pH 7.4) containing 0.1-0.2 wt.% lithium phenyl-2, 4, 6-trimethylbenzoylphosphinate (LAP) photoinitiator;

mixing a solution of ODEX (e.g. 50-60% w/v in PBS) with a solution of DHA-HA or DHA-MeHA (1-2% w/v) to obtain a dynamic acylacylhydrazone bond cross-linked hydrogel; and
Photopolymerizing the dynamic acylhydrazone bond cross-linked hydrogel by irradiation, to obtain the dual-crosslinked MeHA/DHA-HA/ODEX or DHA-MeHA/ODEX hydrogel.

In some embodiments, 0.1 ml solutions of ODEX (50-60% w/v in PBS (Invitrogen)) were pipetted into 0.9 mL DHA-HA or DHA-MeHA (1-2% w/v) contained in a cylindrical mold made from a truncated 1-mL syringe. The mixture was vigorously stirred using the pipet tip and then put on a shaker for gelation which typically took about 0.5-2 min. The resulting round hydrogel disks were exposed to 10 mW $cm^{-2}$ 405 nm blue light for 1-3 min on each side. Disk shaped hydrogels (~4 mm width, ~1 mm height) were cored out of the gel slab and immersed and equilibrated with PBS for 12-16 h.
Therefore, the present invention provides a novel, mild dual-crosslinked hydrogel which has advantageous properties like rapid self-healing (although worse than the hydrogel precursor of the invention), and good cytocompatibility. In addition, the hydrogel precursor, i.e., dynamic acylhydrazone bond cross-linked hydrogel is injectable and has the property of shear-thinning before irradiation; the hydrogel can self-heal immediately after shear induced flow and can be stabilized through light-initiated radical polymerization of methacrylate functional groups to tune gel mechanics and degradation kinetics.
Other advantages of the present invention would be apparent for a person skilled in the art upon reading the specification.

## Brief Description of Drawings

Figure 1 shows $^1H$ NMR spectra of functionalized HA and dextran-aldehyde, including (a) MeHA, (b) DHA-HA, (c) DHA-MeHA and (d) ODEX. MeHA, DHA-MeHA and ODEX samples were prepared during Example 1 and DHA-HA sample was prepared during Example 4.

Figure 2 shows the shear-thinning (a) upon the continuous step shear rates between 1 $s^{-1}$ to 10 $s^{-1}$ and self-healing

(b) upon alternative shear rate between 0.1 $s^{-1}$ and 1 $s^{-1}$ at 37 °C of the dynamic acylhydrazone bond cross-linked hydrogel prepared during Example 1.

Figure 3 shows the representative curves of storage (G') and loss (G") moduli during irradiation of:

the dynamic acylhydrazone bond cross-linked hydrogel with dynamic-crosslinking only prepared during Example 1(Figure 3a);
the photo-crosslinked HA-based hydrogel without ODEX treatment prepared in Comparative Example 1 with light irradiation (10 $mW/cm^2$) (Figure 3b); and
the dynamic acylhydrazone bond cross-linked hydrogel with ODEX treatment prepared in Example 1 (Figure 3c). The in-situ crosslinking process involved both dynamic-crosslinking and light exposure. After light irradiation, double-bond crosslinking formed in the dynamic acylhydrazone bond cross-linked hydrogel and the hydrogel became a dual-crosslinked HA-dextran hydrogel finally prepared in Example 1.

Figure 4 shows degradation kinetics of non-UV-treated hydrogel (dynamic acylhydrazone bond cross-linked hydrogel prepared during Example 1 before irradiation) and UV-treated hydrogel finally prepared in Example 1. For all time points, the percentage of HA release was greater from non-UV-treated hydrogel relative to UV-treated hydrogel ($p<0.01$). Error bars represent standard error of the mean.

Figure 5 shows quantification of cell viability from LIVE/DEAD assay (n=3).

Figure 6 shows gross appearance of defect site at 12 weeks of surgery in control and dual-crosslinked HA-dextran hydrogel prepared in Example 1, where dual-crosslinked HA-dextran hydrogel was in-situ implanted to the right knee joint while the empty defects of left knee joint were acted as control group.

## Detailed description of the invention

The invention is now described in detail by the following examples.

Example 1

Methacrylated HA (MeHA)

Sodium hyaluronate (Evonik Industries AG, 200-500 kDa) was dissolved at in deionized water (DI $H_2O$) to prepare a 1 wt% solution. Methacrylic anhydride (MA; Sigma) was added dropwise (~2.5 mL per g HA) with stirring at 4 °C. The stirring mixture was maintained at pH ~8 by continuously adding 10 mM NaOH for 8 h, followed by reaction overnight at 4 °C and further addition of MA (~1 mL MA per g HA) with pH maintenance for ~4 h. The obtained macromer solution was dialyzed against DI $H_2O$ ($M_W$ cutoff 8,000-14,000) for 3 d, frozen at -80 °C, lyophilized, and stored at -80 °C in powder form. The modification degree of MeHA was 160% according to $^1$H-NMR analysis.

Dihydrazide-MeHA (DHA-MeHA)

MeHA prepared above was dissolved in $H_2O$ at a concentration of 3 mg/mL. To this solution a 30-fold molar excess of adipic dihydrazide was added. The pH of the reaction mixture was adjusted to 6.8 with 0.1 M NaOH/0.1M HCl. 1-Ethyl-3-[3 (dimethylamino)propyl]-carbodiimide (EDC) (192 mg, 1 mmol; Aldrich Chemical Co.) and 1-hydroxybenzotriazole (HOBt) (135 mg, 1 mmol; Aldrich Chemical Co.) was dissolved in dimethylsulfoxide (DMSO)/$H_2O$ (1:1, 1 mL). After mixing, the pH of the reaction was maintained at 6.8 by the addition of 0.1M NaOH and the reaction was allowed to proceed overnight. The pH was subsequently adjusted to 7.0 with 0.1M NaOH and the derivatized HA exhaustively was dialyzed against DI $H_2O$ ($M_W$ cutoff 8,000-14,000) for 3 d, frozen at -80 °C, lyophilized, and stored at -80 °C in powder form. The dihydrazide modification degree of DHA-MeHA was 36% according to $^1$H-NMR analysis.

Dextran-aldehyde (ODEX)

To functionalize dextran (molecular weight: 20 kDa) with aldehyde groups, 5 g dextran was dissolved in water at a concentration of 250 mg/mL. An aqueous solution of sodium periodate (0.3 g dissolved in 5 mL water) was added dropwise, and the reaction mixture was stirred for 2 h at room temperature in the dark. Ethylene glycol was then added to inactivate any unreacted periodate. The solution was purified by exhaustive dialysis against water for 3 days, and the dry product of the dextran-aldehyde was obtained by freeze drying. The modification degree of dextran-aldehyde was

91% according to trinitrobenzene sulfonate assay.

Dual-crosslinked DHA-MeHA/ODEX hydrogel formation

A 2.0 wt.% DHA-MeHA solution was prepared by dissolving DHA-MeHA in PBS (pH 7.4) containing 0.1 wt.% lithium phenyl-2, 4, 6-trimethylbenzoylphosphinate (LAP) photoinitiator (Tokyo Chemical Industry Co., Ltd.). 0.1 ml ODEX (60% w/v prepared by dissolving ODEX in PBS (Invitrogen)) solution was pipetted into 0.9 mL DHA-MeHA (2% w/v in PBS) solution contained in a cylindrical mold made from a truncated 1-mL syringe. The mixture was vigorously stirred using the pipet tip and then put on a shaker for gelation which typically took about 60 s. The resulting round hydrogel disk, which was a dynamic acylhydrazone bond cross-linked hydrogel, was exposed to 10 mW/cm$^2$ 405 nm blue light for 60 s on each side. The disk shaped dual-crosslinked hydrogel (~4 mm width, ~1 mm height) was cored out of the gel slab and immersed and equilibrated with PBS for 12 h. Multiple dual-crosslinked hydrogel samples were prepared in Example 1. The storage modulus of the obtained hydrogel was about 1910 kPa.

Comparative Example 1: DHA-MeHA hydrogel

A 2.0 wt.% solution of DHA-MeHA prepared during Example 1 was prepared in PBS (pH 7.4) containing 0.1 wt.% lithium phenyl-2, 4, 6-trimethylbenzoylphosphinate (LAP) photoinitiator. LAP was chosen due to its aqueous solubility and good cytocompatibility. The mixtures were pipetted between glass slides and exposed to 10 mW/cm$^2$ 405 nm blue light for 1 min on each side. Disk shaped hydrogels (~4 mm width, ~1 mm height) were cored out of the gel slab.
The modulus of the photo-crosslinked DHA-MeHA hydrogel was 3.08 kPa. The determination method was the same as the method described below used for Example 1.

Example 2

MeHA and dihydrazide-MeHA (DHA-MeHA) was prepared according to the same method as Example 1.
Dextran-aldehyde was prepared according to the same method as Example 1.

Dual-crosslinked DHA-MeHA/ODEX hydrogel formation

Dual-crosslinked DHA-MeHA/ODEX hydrogel was prepared according to the same method as Example 1 except that the resulting dynamic acylhydrazone-crosslinked hydrogel was exposed to 10 mW/cm$^2$ 320-500 nm irradiation (comprising 405 nm blue light) instead of 405 nm blue light. The storage modulus of the obtained hydrogel was about 780 kPa. As the irradiation was not done under the optimum irradiation wavelength 405 nm, the storage modulus was lower than that in Example 1.

Example 3

MeHA and dihydrazide-MeHA (DHA-MeHA) was prepared according to the same method as Example 1.
Dextran-aldehyde was prepared according to the same method as Example 1 except that the aqueous solution of sodium periodate used 0.25 g instead of 0.3 g sodium periodate dissolved in 5 mL water. The modification degree of dextran-aldehyde was 86% according to trinitrobenzene sulfonate assay.

Dual-crosslinked DHA-MeHA/ODEX hydrogel formation

A 1 wt.% DHA-MeHA solution was prepared in PBS (pH 7.4) containing 0.2 wt.% lithium phenyl-2, 4, 6-trimethylbenzoylphosphinate (LAP) photoinitiator. 0.1 ml ODEX (60% w/v in PBS) solution was pipetted into 0.9 mL DHA-MeHA (2% w/v in PBS) solution contained in a cylindrical mold made from a truncated 1-mL syringe. The mixture was vigorously stirred using the pipet tip and then put on a shaker for gelation which typically took about 60 s. The resulting round hydrogel disk was exposed to 10 mW/cm 320-500 nm irradiation (comprising 405 nm blue light) for 20 s on each side. The disk-shaped hydrogel (~4 mm width, ~1 mm height) was cored out of the gel slab and immersed and equilibrated with PBS for 12 h. The storage modulus of the obtained hydrogel was about 295 kPa. As the irradiation was not done under the optimum irradiation wavelength 405 nm, the storage modulus was lower than that in Example 1.

Example 4: separate modification to HA by MA and DHA

Methacrylated HA (MeHA) was prepared according to the same method as Example 1.

Dihydrazide-HA (DHA-HA)

Sodium hyaluronate (Evonik Industries AG, 200-500 kDa) was dissolved in $H_2O$ at a concentration of 3 mg/mL. To this solution a 30-fold molar excess of adipic dihydrazide was added. The pH of the reaction mixture was adjusted to 6.8 with 0.1 M NaOH/0.1M HCl. 1-Ethyl-3-[3 (dimethylamino)propyl]-carbodiimide (EDC) (192 mg, 1 mmol) and 1-hydroxybenzotriazole (HOBt) (135 mg, 1 mmol) was dissolved in dimethylsulfoxide (DMSO)/$H_2O$ (1:1, 1 mL). After mixing, the pH of the reaction was maintained at 6.8 by the addition of 0.1M NaOH and the reaction was allowed to proceed overnight. The pH was subsequently adjusted to 7.0 with 0.1M NaOH and the derivatized HA exhaustively was dialyzed against DI $H_2O$ ($M_W$ cutoff 8,000-14,000) for 3 d, frozen at -80 °C, lyophilized, and stored at -80 °C in powder form.

Dextran-aldehyde was prepared according to the same method as Example 1 except that the aqueous solution of sodium periodate used 0.5 g instead of 0.3 g sodium periodate dissolved in 5 mL water. The modification degree of dextran-aldehyde was 95% according to trinitrobenzene sulfonate assay.

Dual-crosslinked DHA-HA/MeHA/ODEX hydrogel formation

Dynamic acylhydrazone bond cross-linked hydrogel was prepared by mixing reactive DHA-HA, MeHA and dextran-aldehyde polymer solutions containing 0.2 wt.% lithium phenyl-2, 4, 6-trimethylbenzoylphosphinate (LAP) photoinitiator. Specifically, 0.1 ml ODEX (50% w/v in PBS) solution was pipetted into 0.9 mL DHA-HA/MeHA (1:1, 2% w/v in PBS, which was prepared by mixing 2% w/v DHA-HA in PBS with 2% w/v MeHA in PBS) solution containing 0.2 wt.% LAP photoinitiator contained in a cylindrical mold made from a truncated 1-mL syringe. The mixture was vigorously stirred using the pipet tip and then put on a shaker for gelation which typically took about 60 s. The resulting round hydrogel disk was exposed to 10 mW/cm 320-500 nm irradiation (comprising 405 nm blue light) for 20 s on each side. The resulting round hydrogel disk was ejected from the cylindrical mold, and immersed and equilibrated with PBS for 12 h. The storage modulus of the obtained hydrogel was about 202 kPa. As the irradiation was not done under the optimum irradiation wavelength 405 nm, the storage modulus was lower than that in Example 1.

Example 5: separate modification to HA by MA and DHA

Methacrylated HA (MeHA) was prepared according to the same method as Example 1.

Dihydrazide-HA (DHA-HA) was prepared according to the same method as Example 4.

Dextran-aldehyde was prepared according to the same method as Example 1.

Dual-crosslinked DHA-HA/MeHA/ODEX hydrogel formation

Dynamic acylhydrazone bond cross-linked hydrogel was prepared by mixing reactive DHA-HA, MeHA and dextran-aldehyde polymer solutions containing 0.1 wt.% lithium phenyl-2, 4, 6-trimethylbenzoylphosphinate (LAP) photoinitiator. Specifically, 0.1 ml ODEX (55% w/v in PBS) solution was pipetted into 0.9 mL DHA-HA/MeHA (1:1, 1% w/v in PBS, which was prepared by mixing 1% w/v DHA-HA in PBS with 1% w/v MeHA in PBS) solution containing 0.2 wt.% LAP photoinitiator contained in a cylindrical mold made from a truncated 1-mL syringe. The mixture was vigorously stirred using the pipet tip and then put on a shaker for gelation which typically took about 60 s. The resulting round hydrogel disk was exposed to 10 mW/cm 320-500 nm irradiation (comprising 405 nm blue light) for 30 s on each side. The disk shaped dual-crosslinked hydrogel (~4 mm width, ~1 mm height) was cored out of the gel slab and immersed and equilibrated with PBS for 12 h. The storage modulus of the obtained hydrogel was about 185 kPa. As the irradiation was not done under the optimum irradiation wavelength 405 nm, the storage modulus was lower than that in Example 1.

Characterization of the dual-crosslinked hydrogel

The MeHA, DHA-MeHA, and ODEX samples prepared during Example 1 and DHA-HA sample prepared during Example 4 were analyzed by using NMR spectrometer. The results were shown in Figure 1.

The chemical structure of functional HA derivatives including MeHA, DHA-HA, DHA-MeHA and dextran-aldehyde was determined on a Bruker Advance III spectrometer using $D_2O$ as the solvent. Proton-NMR of the modified HA revealed distinct methylene (HA 6.1 and 5.6 ppm). The degree of methacrylate modification was determined from the relative integrations of the methacrylate (1H each) to the HA backbone protons (10H).

As shown in Figure 1a, for the MeHA, the signals of vinyl protons clearly appeared at 5.6 and 6.1 ppm. According to the peak areas of 5.6 ppm and 6.1 ppm relative to the sugar ring of HA (3.1-3.8 ppm, 10H) in the MeHA spectrum, the degree of methacrylation was about 160%.

The chemical structure of DHA-HA was shown in Figure 1b, the apparent degree of modification calculated by the ratio of the protons of adipic (4H, $CH_2CH_2$, 1.35-1.55 ppm) to the sugar ring of HA (3.1-3.8 ppm, 10H) was about 36%.

In the spectrum of the DHA-MeHA, the vinyl protons (5.6 ppm, 6.1 ppm) and adipic protons (4H, $CH_2CH_2$, 1.35-1.55 ppm) were shown in Figure 1c, where the degree of methacrylation was 160%, and the degree of hydrazide groups modification was 32%.

In the spectra of dextran-aldehyde, ODEX presents several additional peaks in the range of 5.0-5.8 ppm, which were assigned to the protons from the hemiacetal structures (Figure 1d).

Furthermore, the rheology tests (moduli, shear-thinning and self-healing) as shown below and in Figure 3 confirmed that the desired dual-crosslinking structure were formed in the dynamic acylhydrazone bond cross-linked hydrogel prepared during Example 1.

Figure 3 (a) indicated that dynamic acylhydrazone bond were formed in the dynamic acylhydrazone bond cross-linked hydrogel prepared during Example 1.

Figure 3 (b) indicated that when irradiated, the dihydrazide-MeHA (DHA-MeHA) prepared during Comparative Example 1 may form double-bond crosslinking due to the existence of double-bonds in the methacrylation in the modified HA.

Figure 3 (c) indicated that when irradiated, dual-crosslinking were formed in the dual-crosslinked HA-dextran hydrogel prepared in Example 1.

<u>Performance test of hydrogel</u>

The hydrogels prepared during Example 1 were analyzed according to the methods as follow. The other hydrogels prepared in the description were analyzed according to the same methods.

Moduli, shear-thinning and self-healing:

Moduli, shear-thinning and self-healing of HA-dextran hydrogels prepared during Example 1 including the dynamic acylhydrazone bond cross-linked hydrogel and the dual-crosslinked DHA-MeHA/ODEX hydrogel were assessed with shear rate sweeps and time sweeps using a DHR-2 rheometer (TA Instruments, Delaware, United States) with a quartz plate connected to a blue light source. A plate geometry with a solvent trap, 20 mm diameter, and 500 $\mu$m gap distance was used. Dynamic acylhydrazone bond cross-linked HA-dextran hydrogels prepared during Example 1 were formed by homogenously mixing together HA/dextran components, and loaded onto the rheometer. Shear rate swept between 0.1 to 10 $s^{-1}$ was tested at 37 °C. Time sweeps were performed for shear recovery experiments. Hydrogels were deformed using 1 $s^{-1}$ and allowed to recover at 0.1 $s^{-1}$ shear rate at 37 °C. To measure the response of rheological properties to photopolymerization, in situ polymerization was performed with in-situ dynamic crosslinking with 405 nm wavelength irradiation at 10 $mW/cm^2$ light intensity using a dental lamp attached to a light guide for different formulations for 5-10 min via a light-curing stage during oscillatory time sweeps at a frequency of 1 Hz and a strain of 0.5%. Experiments were repeated for a minimum of three times, and representative data was presented.

As shown in Figure 2a, the viscosity of the dynamic acylhydrazone bond cross-linked hydrogel continuously decreased with shear rate increased from 1 $s^{-1}$ to 10 $s^{-1}$, which suggests that such hydrogels exhibit shear-thinning property suitable for easy injection. Moreover, these hydrogels were able to self-heal after shear injection. As shown in Figure 2a, at 1 $s^{-1}$ shear rate, the viscosity of the dynamic acylhydrazone bond cross-linked HA-dextran hydrogel abruptly decreased from 418 Pa·s to 82 Pa·s. In contrast, as the shear rate was jumped to 0.1 $s^{-1}$, the viscosity rapidly recovered to its original value. These values demonstrate that the dynamic acylhydrazone bond cross-linked HA-dextran hydrogels self-healed rapidly into hydrogels and the methacrylate functionality did not interfere with the shear-thinning and self-healing properties of the dynamic hydrogels.

The representative curves of storage (G') and loss (G") moduli of the hydrogels prepared during Example 1 and Comparative Example 1 were shown in Figure 3. The storage modulus (G') and the loss modulus (G") of the hydrozide-crosslinking DHA-MeHA/ODEX were firstly monitored with an oscillatory time sweep.

Figure 3a shows the dynamic acylhydrazone bond crosslinking step during Example 1, the injectable liquid-like property of the precursor (before dynamic acylhydrazone bond crosslinking) was shown from the larger G" than G'. After crosslinking of 25 s, the injectable precursor was converted into a gel (dynamic acylhydrazone bond cross-linked HA-dextran hydrogel), where the G' was larger than G". Allowing further crosslinking, the G' of dynamic DHA-MeHA/ODEX hydrogels reached ~40 Pa at about 1200 s of crosslinking. These values suggested that the dynamic acylhydrazone bond cross-linked DHA-MeHA/ODEX hydrogels were soft and weak, which were beneficial for injection.

Figure 3b shows the formation of DHA-MeHA hydrogel prepared during Comparative Example 1. Upon exposure to free-radical generating irradiation using a 405 nm blue-light, the precursor (DHA-MeHA without ODEX component) exhibit increased in both G' and G", and was rapidly converted into a hydrogel (< 30 s), as reflected by the increase in G' over the G". Allowing crosslinking to proceed to completion, the G' of DHA-MeHA hydrogels reached as high as ~3.08 kPa at about 180 s of irradiation.

Figure 3c shows the formation of dual-crosslinked DHA-MeHA hydrogel prepared during Example 1. As shown

in Figure 3(c), the DHA-MeHA/ODEX hydrogels were firstly crosslinked by dynamic hydrazone and subsequently strengthened via chemical crosslinking. The dual-crosslinked DHA-MeHA/ODEX hydrogels resulted in a more rigid viscoelastic solid. The G' of the dual-crosslinked DHA-MeHA/ODEX hydrogels reached as high as ~1910 kPa at about 600 s of irradiation, which was much higher than both of the DHA-MeHA/ODEX hydrogels without irradiation (dynamic acylhydrazone bond cross-linked HA-dextran hydrogel) and the DHA-MeHA without ODEX components, due to dynamic acylhydrazone crosslinking and chemical crosslinking between methacrylate groups.

Chemical crosslinking can be terminated by simply removing irradiation, and the extent of chemical crosslinking can be tuned by adjusting irradiation exposure time. The G' of such dual-crosslinking hydrogel could be adjust from 0.1~1000 kPa, which was beyond expectation and very surprising for a person skilled in the art.

Degradation kinetics:

To quantitatively assess degradation kinetics, HA-dextran hydrogels prepared during Example 1, including the dynamic acylhydrazone bond cross-linked hydrogel and the dual-crosslinked DHA-MeHA/ODEX hydrogel (8 mm diameter, 3 mm thickness) were incubated in separate wells of a 24-well plate containing 5 U/mL exogenous hyaluronidase in 1.0 mL PBS on an orbital shaker at 37 °C. The solutions were refreshed every 48 hours until day 14. Hydrogel samples were removed from the cultures after 1-2 d, washed with distilled water, and lyophilized in a freeze dryer at -65 °C for 3 days and weighed. The extent of degradation was estimated from the weight loss of the polymer based on the following equation:

$$W=(W_o-W_d)/W_o*100\%$$

Where $W_o$ is the original weight of the hydrogel samples, and $W_d$ is the weight of dry sample after being degraded by hydrolysis in PBS.

Degradation kinetics of the dynamic acylhydrazone bond cross-linked hydrogels and dual-crosslinked hydrogels after irradiation prepared during Example 1 in the presence of 5 U/ml exogenous hyaluronidase for 14 d were shown in Figure 4. Dynamic acylhydrazone bond cross-linked hydrogels exhibited rapid HA release, whereas irradiated hydrogels exhibited little HA release, which suggested that secondary polymerization inhibited the degradation of the hydrogels.

Cell viability test:

Bone marrow stromal cells (BMSCs) were isolated from neonatal SD rats. Cells were cultured in low-glucose Dubecco's Modified Eagle Medium (DMEM, Gibco) supplemented with 10% fetal bovine serum (FBS, Gibco). These cells were located in humidified incubator at 37 °C with a 5% $CO_2$ atmosphere. After 3 days, non-adherent cells were removed and fresh media was added. The cells were passaged upon almost confluence. Only three-passage BMSCs were used for hydrogel studies.

The same preparation method as Example 1 were performed except that cells were incorporated into the hydrogel in the stage of "Dual-crosslinked DHA-MeHA/ODEX hydrogel formation". Single cell suspensions of BMSCs were mixed with DHA-MeHA and ODEX solutions prepared in Example 1. The final concentration of encapsulated BMSCs in the Dual-crosslinked DHA-MeHA/ODEX hydrogel prepared in Example 1 was $1 \times 10^6$ cells $mL^{-1}$. The hydrogels (50 $\mu$L each) encapsulated with BMSCs were incubated a 24-well culture plate at 37 °C in 5% $CO_2$ with medium change every two days. Cell viability and proliferation of the encapsulated hASCs were determined by LIVE/DEAD assays according to the manufacturer's protocol (Biovision). For quantitative analysis of LIVE/DEAD result, images were taken randomly from three hydrogel samples at 1, 7 and 14 d after encapsulation using a Leica inverted microscope. The cell viability was defined as the ratio between the number of live cells and the total cell number in the field.

The cell viability was demonstrated by using LIVE/DEAD assay (n=3). As shown in Figure 5, the viable stem cells remained above 90 % after 14 days, which indicated the dual-crosslinked hydrogel are cytocompatible and the photo-irradiation does not harm the cells.

Cartilage tissue regeneration

Twelve healthy mature male New Zealand white rabbits weighing around 2.5-3 kg were used for cartilage repair experiment. The full thickness cartilage defects with ~4 mm diameter were created on each knee joint. The empty defects of left cartilage were acted as control group. All surgical procedures were performed under aseptic conditions. The rabbits were anaesthetized by using chloral hydrate (0.15 g/kg body weight). The hind limb was shaved, prepped with betadine and 70% alcohol. A parapatellar, longitudinal incision was made and the patella was luxated medially exposing

the femoropatellar groove. Using a dental drill of 3.5 mm diameter, a circular defect (~4 mm in diameter and ~4 mm in depth) was created at the femoro patellar groove by increasing the defect size sequentially to full osteochondral thickness. The drilling was done under continuous irrigation with saline solution for cooling and removing residual tissue while creating the defect. A sterile gauss was used to blot blood at the defect and to obtain homeostasis. Aseptically prepared dynamic acylhydrazone bond cross-linked hydrogel hydrogels prepared during Example 1 (without cells) of each rabbit group were loaded into a syringe. The defect was filled with injectable hydrogels (150 $\mu$L) using 35G needle and then was exposed to 10 mW $cm^{-2}$ 405 nm blue light for 180 s. The patella was repositioned, followed by suturing the muscle and skin separately. The osteochondral defect was also created in contralateral limb by repeating the same procedure. Bilateral surgery was performed in the articulating knee joints and the approach was identical for all rabbits. Animals were moved to their cages after they recovered from anesthesia. Post and pre-surgical care was taken by intra-muscular administration of penicillin (400,000 units). At 12 weeks, animals were euthanized by carbon dioxide inhalation and the femoral end of knee joints was dissected for further analysis.

As shown in Figure 6, after 12 weeks, the hydrogel injected groups exhibited closure of defect with no adverse degeneration of tissue when compared control groups. Moreover, more deposition of newly formed tissue was observed in animals treated with hydrogel group.

As used herein, terms such as "comprise(s)" and the like as used herein are open terms meaning 'including at least' unless otherwise specifically noted. Where a numerical limit or range is stated, the endpoints are included. Also, all values and subranges within a numerical limit or range are specifically included as if explicitly written out.

The above description is presented to enable a person skilled in the art to make and use the invention, and is provided in the context of a particular application and its requirements.

## Claims

**1.** A process to prepare a dual-crosslinked hydrogel, comprising the following steps:

1) functionalizing a glycosaminoglycan such as hyaluronic acid with a methacrylate on hydroxyl group and with a dihydrazide on carboxyl group to obtain a methacrylate and hydrazide group functionalized glycosaminoglycan, which can be either a glycosaminoglycan with both methacrylate and hydrazide group modification, or a glycosaminoglycan with methacrylate modification only and a glycosaminoglycan with hydrazide group modification only, wherein the degree of methacrylation is 5-200%, preferably 85-165%, and the degree of hydrazide group modification is 8-70%, preferably 10-65%, more preferably 10-40%; oxidizing a second-polysaccharide such as dextran to obtain a second-polysaccharide with aldehyde groups, the degree of aldehyde modification in the second-polysaccharide is 10-95%, preferably 85~95%;
2) crosslinking the methacrylate and hydrazide group functionalized glycosaminoglycan and the second-polysaccharide-aldehyde in an aqueous solvent, such as phosphate buffer saline or a cell culture medium, to form a dynamic acylhydrazone bond cross-linked hydrogel; and
3) photopolymerizing the dynamic acylhydrazone bond cross-linked hydrogel by irradiation under the presence of a photoinitiator.

**2.** The process of claim 1, wherein the glycosaminoglycan is water soluble and is selected from glycosaminoglycans that may be modified with a methacrylate on a hydroxyl group and with a dihydrazide on a carboxyl group; preferably selected from the group consisting of hyaluronic acid, chondroitin sulfate, dermatan sulfate, heparan sulfate and heparin, more preferably is hyaluronic acid, especially hyaluronic acid with a molecular weight of 50-2000 kDa, preferably 200-1000 kDa.

**3.** The process of claim 1, wherein the second-polysaccharide is a water soluble polysaccharide, and comprises a 1,4-linkage or a 1,6-linkage, and comprises an ortho-hydroxyl on C2, C3 or C4 of the sugar ring; preferably is selected from the group consisting of glucans such as $\beta$-1,3/1,6-glucan, $\alpha$-1,6-glucan with $\alpha$-1,3-branches, $\alpha$-1,6- glucan, $\alpha$ -1,4/1,6-glucan, alginate and pectin, especially dextran, laminarin ($\beta$-1,3- and $\beta$-1,6-glucan), or water soluble cellulose derivatives; more preferably is dextran, especially dextran with a molecular weight of 10-1000kDa, such as 10-100kDa, 10~70 kDa, preferably 10-20 kDa.

**4.** The process of claim 1, wherein the methacrylate is selected from hydrolyzable methacrylates; preferably is selected from the group consisting of methacrylic anhydride, 2-aminoethyl methacrylate hydrochloride, and glycidyl methacrylate; more preferably is methacrylic anhydride or glycidyl methacrylate.

**5.** The process of claim 1, wherein the dihydrazide is water-soluble and is a dihydrazide that may, after hydrazide

group modification of the glycosaminoglycan, further react with an aldehyde group to form an acylhydrazone bond; preferably is selected from the group consisting of adipic dihydrazide, ethanedihydrazide, oxalyldihydrazide, and dodecanedioic dihydrazide.

**6.** The process of claim 1, wherein the concentration of modified glycosaminoglycan such as modified hyaluronic acid is 0.5-3wt.%, preferably 1~2wt.%, the concentration of second-polysaccharide-aldehyde such as dextran-aldehyde is 4-8wt.%, preferably 4~6wt.% based on the total weight of the reaction mixture in step 2).

**7.** The process of claim 1, wherein the irradiation is 405 nm blue light, for example at a light intensity of 6~10 $mW/cm^2$ for 1~3 min.

**8.** The process of claim 1, wherein cells are incorporated into the dual-crosslinked hydrogel by impregnating the dynamic acylhydrazone bond cross-linked hydrogel in a cell suspension; or by adding cells into the reaction mixture before forming the dynamic acylhydrazone bond cross-linked hydrogel, preferably by mixing cells with the functionalized glycosaminoglycan and/or the second-polysaccharide-aldehyde.

**9.** A dual-crosslinked hydrogel prepared according to the process of any one of claims 1-8.

**10.** A hydrogel precursor to prepare a dual-crosslinked hydrogel, which is prepared by the following steps:

1) functionalizing a glycosaminoglycan such as hyaluronic acid with methacrylate on hydroxyl group and with a dihydrazide on carboxyl group to obtain a methacrylate and hydrazide group functionalized glycosaminoglycan, which can be either a glycosaminoglycan with both methacrylate and hydrazide group modification, or a glycosaminoglycan with methacrylate modification only and a glycosaminoglycan with hydrazide group modification only, wherein the degree of methacrylation is 5-200%, preferably 85-165%, and the degree of hydrazide group modification is 8-70%, preferably 10-65%, more preferably 10-40%; oxidizing a second-polysaccharide such as dextran to obtain a second-polysaccharide with aldehyde groups, the degree of aldehyde modification in the a second-polysaccharide is 10-95%, preferably 85~95%; and
2) crosslinking the methacrylate and hydrazide group functionalized glycosaminoglycan and the second-polysaccharide-aldehyde in an aqueous solvent, such as phosphate buffer saline (PBS) or a cell culture medium, to form a dynamic acylhydrazone bond cross-linked hydrogel.

**11.** Use of the dual-crosslinked hydrogel of claim 9 or the hydrogel precursor of claim 10 in tissue engineering, bioapplications such as drug delivery system, biosensors and soft tissue filling agents, etc., or as a carrier of at least one active ingredient such as cell.

**Patentansprüche**

**1.** Verfahren zur Herstellung eines zweifach quervernetzten Hydrogels, wobei es die folgenden Schritte umfasst:

1) Versehen eines Glycosaminoglycans wie etwa Hyaluronsäure mit einer Methacrylatfunktion an einer Hydroxylgruppe und mit einer Dihydrazidfunktion an einer Carboxylgruppe, um ein Glycosaminoglycan zu erhalten, welches über eine Methacrylat- und eine Hydrazidfunktion verfügt, wobei es sich entweder um ein Glycosaminoglycan, welches mit einer Methacrylat- oder einer Hydrazidgruppe modifiziert ist, oder um ein Glycosaminoglycan, welches nur mit einer Methacrylatgruppe modifiziert ist, und um ein Glycosaminoglycan handeln kann, welches nur mit einer Hydrazidgruppe modifiziert ist, wobei der Grad der Methacrylierung 5 bis 200 %, vorzugsweise 85 bis 165 % beträgt und der Grad der Hydrazidgruppenmodifizierung 8 bis 70 %, vorzugsweise 10 bis 65 %, insbesondere 10 bis 40 % beträgt; Oxidieren eines zweiten Polysaccharids wie etwas eines Dextrans, um ein zweites Polysaccharid mit Aldehydgruppen zu erhalten, wobei das Ausmaß der Aldehydmodifizierung bei dem zweiten Polysaccharid 10 bis 95 %, vorzugsweise 85 bis 95% beträgt;
2) Quervernetzen des mit funktionellen Methacrylat- und Hydrazidgruppen versehenen Glycosaminoglycans und des zweiten Polysaccharid-Aldehyds in einem wässrigen Lösungsmittel, wie etwa einer phosphatgepufferten Kochsalzlösung oder einem Zellkulturmedium, um ein dynamisches quervernetztes Hydrogel mit Acylhydrazon-Bindungen zu bilden; und
3) Photopolymerisieren des dynamischen quervernetzten Hydrogels mit Acylhydrazon-Bindungen durch Bestrahlen in Gegenwart eines Photoinitiators.

2. Verfahren nach Anspruch 1, wobei das Glycosaminoglycan wasserlöslich ist und aus den Glycosaminoglycanen ausgewählt ist, die an einer Hydroxylgruppe mit einem Methacrylat und and einer Carboxylgruppe mit einem Dihydrazid modifiziert werden können; wobei es vorzugsweise aus der Gruppe ausgewählt ist, die aus Hyaluronsäure, Chondroitinsulfat, Dermatansulfat, Heparansulfat und Heparin besteht, wobei es sich insbesondere um Hyaluronsäure mit einem Molekulargewicht von 50 bis 2000 kDa, vorzugsweise 200 bis 1000 kDa handelt.

3. Verfahren nach Anspruch 1, wobei es sich bei dem zweiten Polysaccharid um ein wasserlösliches Polysaccharid handelt und es eine 1,4-Verknüpfung oder eine 1,6-Verknüpfung umfasst, und es ein ortho-Hydroxyl am C2, C3 oder C4 des Zuckerrings umfasst; wobei es vorzugsweise aus der Gruppe ausgewählt ist, die aus Glucanen wie etwa β-1,3/1,6-Glucan, α-1,6-Glucan mit α-1,3-Verzweigungen, α-1,6-Glucan, α-1,4/1,6-Glucan, Alginat und Pectin, insbesondere Dextran, Laminarin (β-1,3- und β-1,6-Glucan) oder wasserlöslichen Cellulosederivaten besteht; wobei es sich insbesondere um Dextran mit einem Molekulargewicht von 10 bis 1000 kDa, wie etwa 10 bis 100 kDa, 10 bis 70 kDa, vorzugsweise 10 bis 20 kDa handelt.

4. Verfahren nach Anspruch 1, wobei das Methacrylat aus hydrolysierbaren Methacrylaten ausgewählt ist; wobei es vorzugsweise aus der Gruppe ausgewählt ist, die aus Methacrylsäureanhydrid, 2-Aminoethylmethacrylat-Hydrochlorid und Glycidylmethacrylat besteht; wobei es sich insbesondere um Methacrylsäureanhydrid oder Glycidylmethacrylat handelt.

5. Verfahren nach Anspruch 1, wobei das Dihydrazid wasserlöslich ist, und es sich um ein Dihydrazid handelt, das nach der Hydrazidgruppenmodifizierung des Glycosaminoglycans weiterhin mit einer Aldehydgruppe reagieren kann, um eine Acylhydrazon-Bindung zu bilden; wobei es vorzugsweise aus der Gruppe ausgewählt ist, welche aus Adipinsäure-Dihydrazid, Ethandihydrazid, Oxalyldihydrazid und Dodecandisäure-Dihydrazid besteht.

6. Verfahren nach Anspruch 1, wobei die Konzentration an modifiziertem Glycosaminoglycan wie etwa modifizierter Hyaluronsäure 0,5 bis 3 Gew.-%, vorzugsweise 1 bis 2 Gew.-% beträgt, die Konzentration an zweitem Polysaccharid-Aldehyd 4 bis 8 Gew.-%, vorzugsweise 4 bis 6 Gew.-% beträgt, bezogen auf das Gesamtgewicht der Reaktionsmischung in Schritt 2.

7. Verfahren nach Anspruch 1, wobei die Bestrahlung bei 405 nm mit Blaulicht erfolgt, beispielsweise mit einer Lichtintensität von 6 bis 10 $mW/cm^2$, für 1 bis 3 min.

8. Verfahren nach Anspruch 1, wobei dem zweifach quervernetzten Hydrogel derart Zellen beigefügt werden, dass das dynamische quervernetzte Hydrogel mit Acylhydrazon-Bindungen zur Durchtränkung in eine Zellsuspension gegeben wird; oder dass Zellen zur Reaktionsmischung hinzugefügt werden, bevor sich das dynamische quervernetzte Hydrogel mit Acylhydrazon-Bindungen bildet, vorzugsweise derart, dass Zellen mit dem Glycosaminoglycan, welches mit funktionellen Gruppen versehen ist, und/oder dem zweiten Polysaccharid-Aldehyd vermischt werden.

9. Zweifach quervernetztes Hydrogel, das gemäß dem Verfahren nach einem beliebigen der Ansprüche 1 bis 8 hergestellt wurde.

10. Hydrogel-Vorstufe zur Herstellung eines zweifach quervernetzten Hydrogels, wobei sie mittels der folgenden Schritte hergestellt wird:

1) Versehen eines Glycosaminoglycans wie etwa Hyaluronsäure mit einer Methacrylatfunktion an einer Hydroxylgruppe und mit einer Dihydrazidfunktion an einer Carboxylgruppe, um ein Glycosaminoglycan zu erhalten, welches über eine Methacrylat- und eine Hydrazidfunktion verfügt, wobei es sich entweder um ein Glycosaminoglycan, welches mit einer Methacrylat- oder einer Hydrazidgruppe modifiziert ist, oder um ein Glycosaminoglycan, welches nur mit einer Methacrylatgruppe modifiziert ist, und um ein Glycosaminoglycan handelt, welches nur mit einer Hydrazidgruppe modifiziert ist, wobei der Grad der Methacrylierung 5 bis 200 %, vorzugsweise 85 bis 165 % beträgt und der Grad der Hydrazidgruppenmodifizierung 8 bis 70 %, vorzugsweise 10 bis 65 %, insbesondere 10 bis 40 % beträgt; Oxidieren eines zweiten Polysaccharids wie etwas eines Dextrans, um ein zweites Polysaccharid mit Aldehydgruppen zu erhalten, wobei das Ausmaß der Aldehydmodifizierung bei dem zweiten Polysaccharid 10 bis 95 %, vorzugsweise 85 bis 95% beträgt; und
2) Quervernetzen des mit funktionellen Methacrylat- und Hydrazidgruppen versehenen Glycosaminoglycans und des zweiten Polysaccharid-Aldehyds in einem wässrigen Lösungsmittel, wie etwa einer phosphatgepufferten Kochsalzlösung (PBS) oder einem Zellkulturmedium, um ein dynamisches quervernetztes Hydrogel mit Acylhydrazon-Bindungen zu bilden.

11. Verwendung des zweifach quervernetzten Hydrogels nach Anspruch 9 oder der Hydrogel-Vorstufe nach Anspruch 10 in der Gewebezucht, Bioanwendungen wie etwa Wirkstoffzuführungssystemen, Biosensoren und Weichgewebefüllstoffen usw., oder als Trägerstoff für mindestens einen Wirkbestandteil wie etwa eine Zelle.

## Revendications

1. Procédé de préparation d'un hydrogel à double réticulation, comprenant les étapes suivantes :

   1) fonctionnalisation d'un glycosaminoglycane tel que l'acide hyaluronique avec un méthacrylate sur un groupe hydroxyle et avec un dihydrazide sur un groupe carboxyle pour obtenir un glycosaminoglycane fonctionnalisé par méthacrylate et un groupe hydrazide, qui peut être soit un glycosaminoglycane avec à la fois une modification par méthacrylate et un groupe hydrazide, ou un glycosaminoglycane avec modification par un méthacrylate uniquement et un glycosaminoglycane avec modification par un groupe hydrazide uniquement, le degré de méthacrylation étant de 5 à 200 %, de préférence de 85 à 165 %, et le degré de modification par un groupe hydrazide étant de 8 à 70 %, de préférence de 10 à 65 %, plus préférablement de 10 à 40 % ; oxydation d'un deuxième polysaccharide tel que le dextrane pour obtenir un deuxième polysaccharide avec des groupes aldéhyde, le degré de modification aldéhyde dans le deuxième polysaccharide étant de 10 à 95 %, de préférence de 85 à 95 % ;
   2) réticulation du glycosaminoglycane fonctionnalisé par méthacrylate et un groupe hydrazide et le deuxième polysaccharide-aldéhyde dans un solvant aqueux, tel qu'une solution saline tamponnée par les phosphates ou un milieu de culture de cellules, pour former un hydrogel réticulé par liaison acylhydrazone dynamique ; et
   3) photopolymérisation de l'hydrogel réticulé par liaison acylhydrazone dynamique par irradiation en présence d'un photoamorceur.

2. Procédé selon la revendication 1, dans lequel le glycosaminoglycane est soluble dans l'eau et est choisi parmi les glycosaminoglycanes qui peuvent être modifiés par un méthacrylate sur un groupe hydroxyle et par un dihydrazide sur un groupe carboxyle ; de préférence choisi dans le groupe constitué de l'acide hyaluronique, du sulfate de chondroïtine, du sulfate de dermatane, du sulfate d'héparane et de l'héparine, plus préférablement l'acide hyaluronique, en particulier l'acide hyaluronique ayant un poids moléculaire de 50 à 2 000 kDa, de préférence de 200 à 1 000 kDa.

3. Procédé selon la revendication 1, dans lequel le deuxième polysaccharide est un polysaccharide hydrosoluble, et comprend une liaison 1,4 ou une liaison 1,6, et comprend un ortho-hydroxyle sur C2, C3 ou C4 du cycle du sucre ; est de préférence choisi dans le groupe constitué des glucanes tels que le $\beta$-1,3/1,6-glucane, le $\alpha$-1,6-glucane avec des branches $\alpha$-1,3, le $\alpha$-1,6-glucane, le $\alpha$-1,4/1,6-glucane, l'alginate et la pectine, en particulier le dextrane, la laminarine ($\beta$-1,3- et $\beta$-1,6-glucane), ou des dérivés de cellulose solubles dans l'eau ; de préférence, le dextrane, en particulier un dextrane ayant un poids moléculaire de 10 à 1 000 kDa, tel que 10 à 100 kDa, 10 à 70 kDa, de préférence 10 à 20 kDa.

4. Procédé selon la revendication 1, dans lequel le méthacrylate est choisi parmi les méthacrylates hydrolysables ; est de préférence choisi dans le groupe constitué de l'anhydride méthacrylique, du chlorhydrate de méthacrylate de 2-aminoéthyle et du méthacrylate de glycidyle ; plus préférablement, l'anhydride méthacrylique ou le méthacrylate de glycidyle.

5. Procédé selon la revendication 1, dans lequel le dihydrazide est soluble dans l'eau et est un dihydrazide qui peut, après modification par un groupe hydrazide du glycosaminoglycane, réagir en outre avec un groupe aldéhyde pour former une liaison acylhydrazone ; est de préférence choisi dans le groupe constitué du dihydrazide adipique, de l'éthanedihydrazide, de l'oxalyldihydrazide et du dihydrazide dodécanedioïque.

6. Procédé selon la revendication 1, dans lequel la concentration de glycosaminoglycane modifié tel que l'acide hyaluronique modifié est de 0,5 à 3 % en poids, de préférence de 1 à 2 % en poids, la concentration de deuxième polysaccharide-aldéhyde tel que le dextrane-aldéhyde est de 4 à 8 % en poids, de préférence 4 à 6 % en poids sur la base du poids total du mélange de réaction dans l'étape 2).

7. Procédé selon la revendication 1, dans lequel l'irradiation est une lumière bleue à 405 nm, par exemple à une intensité lumineuse de 6 à 10 $mW/cm^2$ pendant 1 à 3 min.

**8.** Procédé selon la revendication 1, dans lequel des cellules sont incorporées dans l'hydrogel à double réticulation par imprégnation de l'hydrogel réticulé par liaison acylhydrazone dynamique dans une suspension de cellules ; ou par ajout de cellules dans le mélange de réaction avant de former l'hydrogel réticulé par liaison acylhydrazone dynamique, de préférence par mélange de cellules avec le glycosaminoglycane fonctionnalisé et/ou le deuxième polysaccharide-aldéhyde.

**9.** Hydrogel à double réticulation préparé selon le procédé de l'une quelconque des revendications 1 à 8.

**10.** Précurseur d'hydrogel pour préparer un hydrogel à double réticulation, qui est préparé par les étapes suivantes :

1) fonctionnalisation d'un glycosaminoglycane tel que l'acide hyaluronique avec un méthacrylate sur un groupe hydroxyle et avec un dihydrazide sur un groupe carboxyle pour obtenir un glycosaminoglycane fonctionnalisé par méthacrylate et un groupe hydrazide, qui peut être soit un glycosaminoglycane avec à la fois une modification par méthacrylate et un groupe hydrazide, ou un glycosaminoglycane avec modification par un méthacrylate uniquement et un glycosaminoglycane avec modification par un groupe hydrazide uniquement, le degré de méthacrylation étant de 5 à 200 %, de préférence de 85 à 165 %, et le degré de modification par un groupe hydrazide étant de 8 à 70 %, de préférence de 10 à 65 %, plus préférablement de 10 à 40 % ; oxydation d'un deuxième polysaccharide tel que le dextrane pour obtenir un deuxième polysaccharide avec des groupes aldéhyde, le degré de modification aldéhyde dans le deuxième polysaccharide étant de 10 à 95 %, de préférence de 85 à 95 % ; et
2) réticulation du glycosaminoglycane fonctionnalisé par méthacrylate et un groupe hydrazide et le deuxième polysaccharide-aldéhyde dans un solvant aqueux, tel qu'une solution saline tamponnée par les phosphates (PBS) ou un milieu de culture de cellules, pour former un hydrogel réticulé par liaison acylhydrazone dynamique.

**11.** Utilisation de l'hydrogel à double réticulation selon la revendication 9 ou du précurseur d'hydrogel selon la revendication 10 dans l'ingénierie tissulaire, des bioapplications telles que des systèmes d'administration de médicament, des biocapteurs et des agents de remplissage de tissus mous, etc., ou en tant que support d'au moins un composant actif tel qu'une cellule.

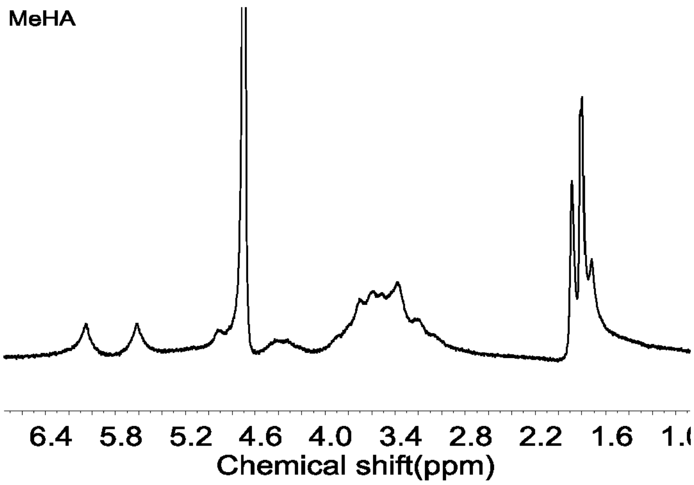


Figure 1(a)

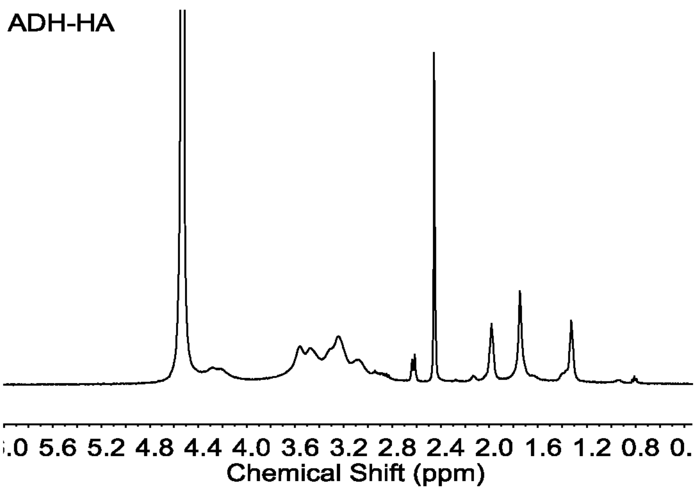


Figure 1(b)

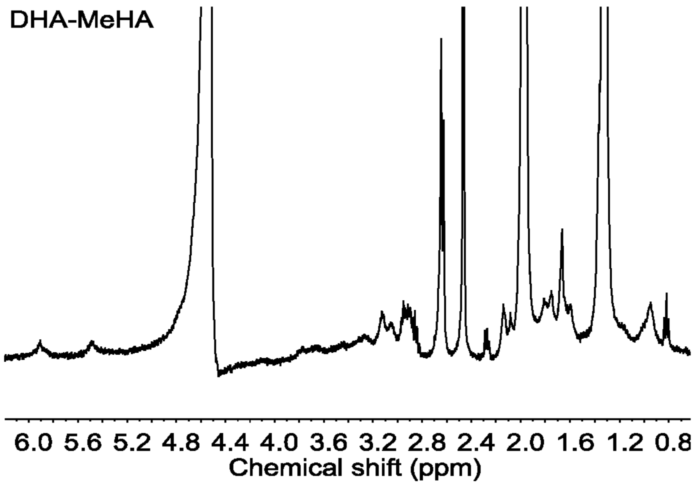


Figure 1(c)

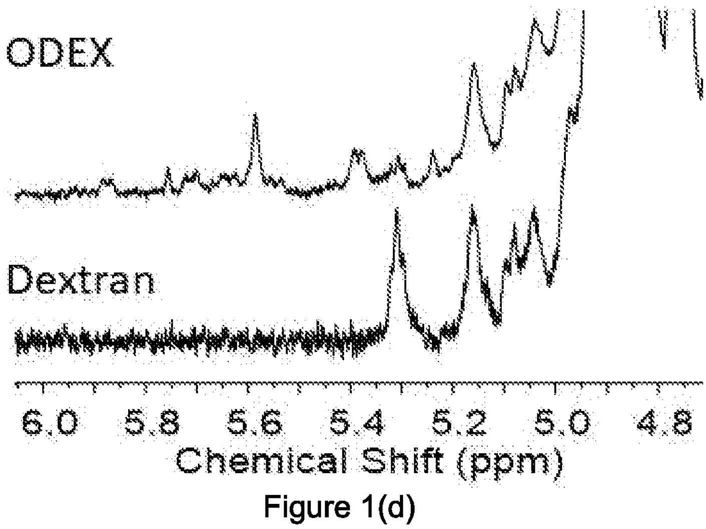


Figure 1(d)

**Figure 1**

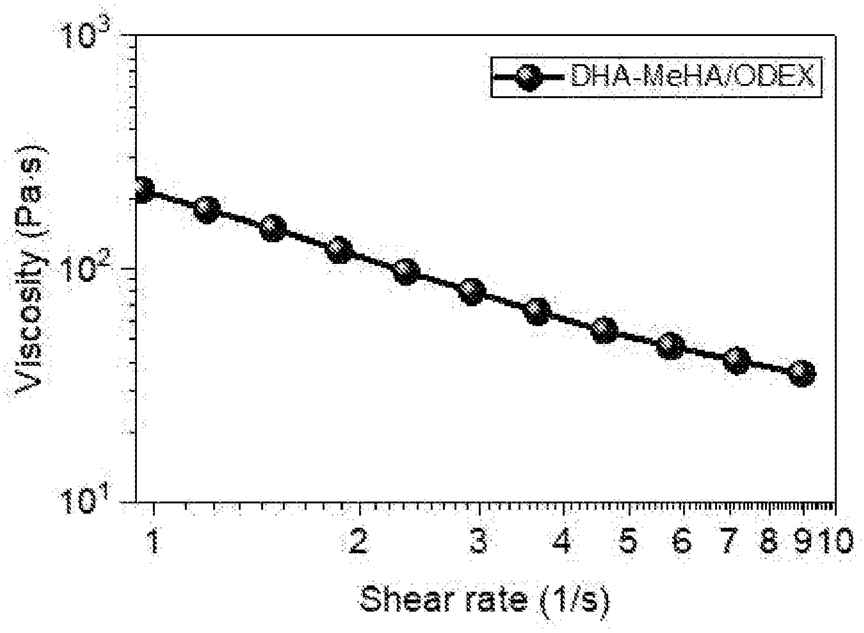


Figure 2 (a)

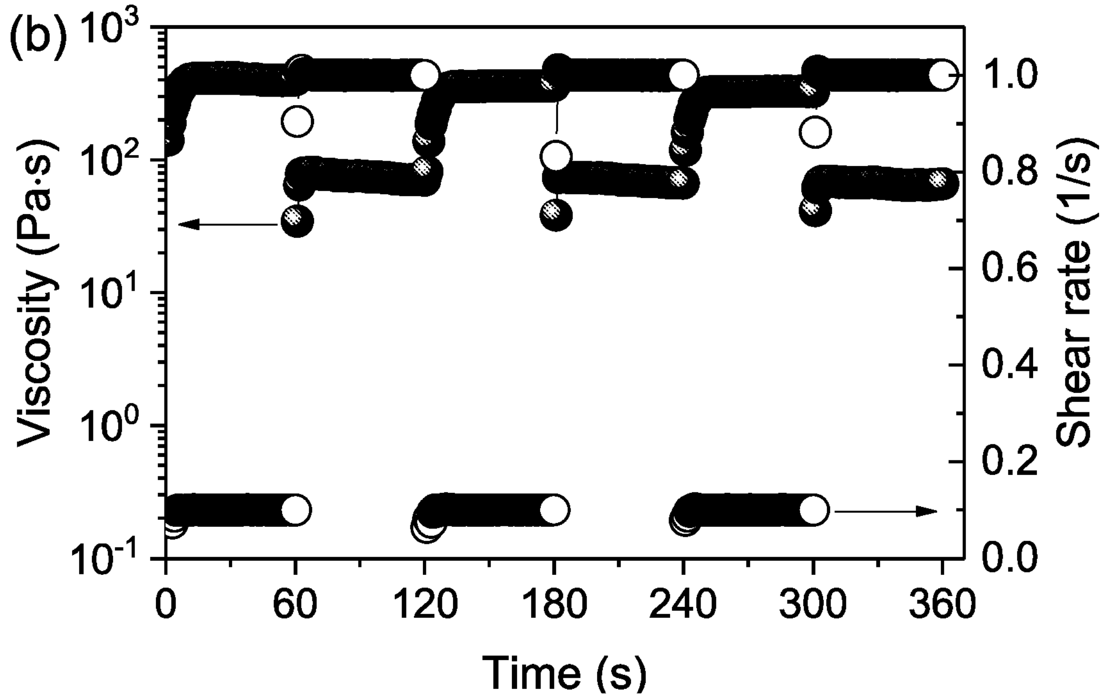


Figure 2 (b)

**Figure 2**

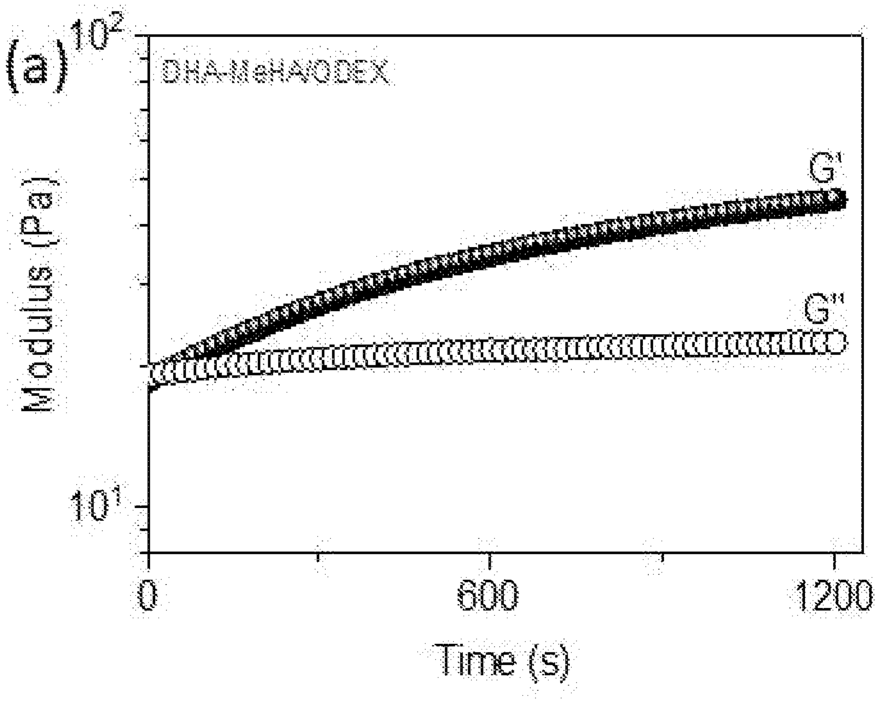


Figure 3 (a)

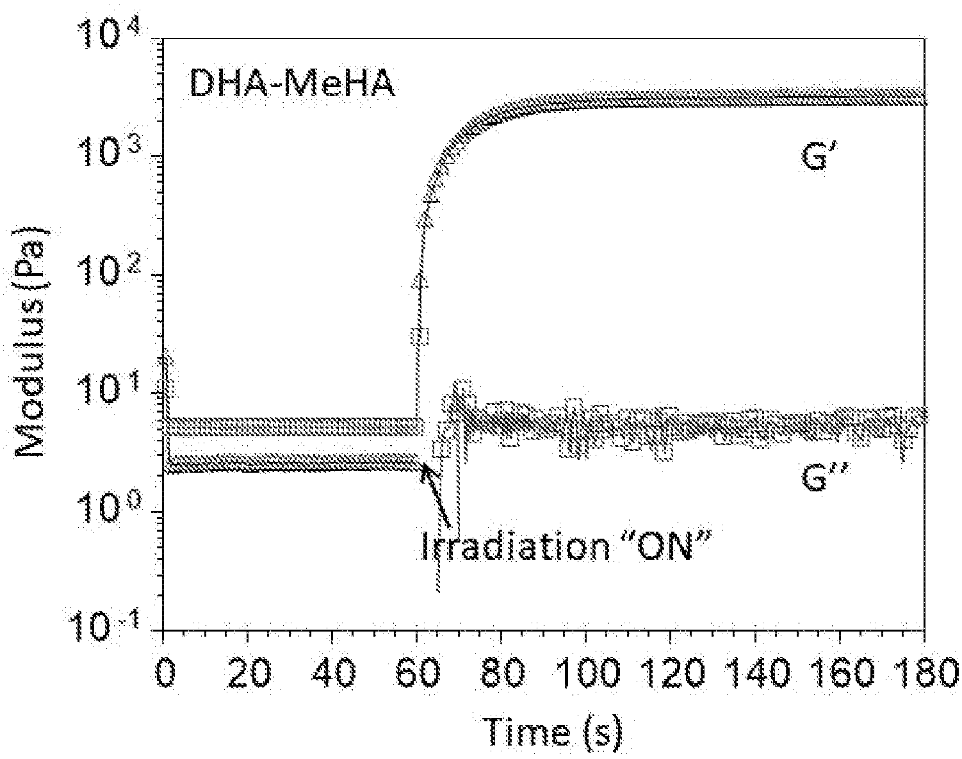


Figure 3 (b)

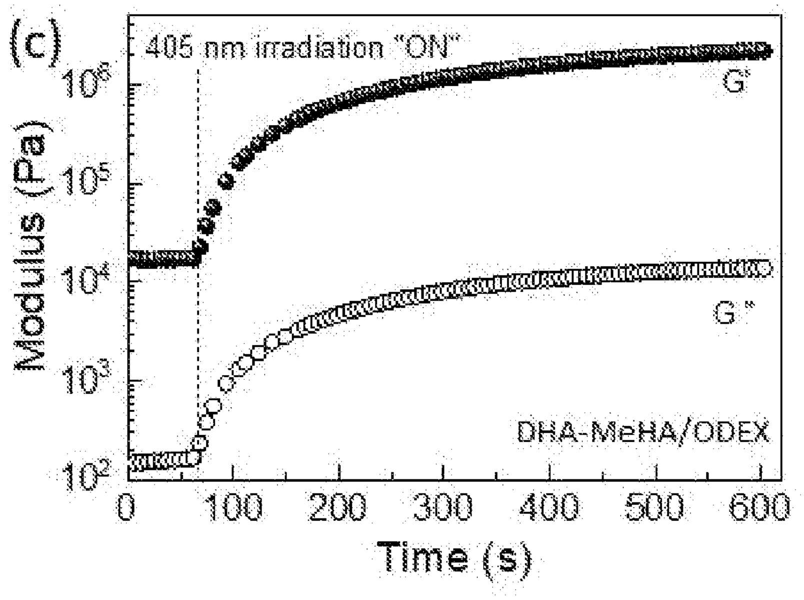


Figure 3 (c)

**Figure 3**

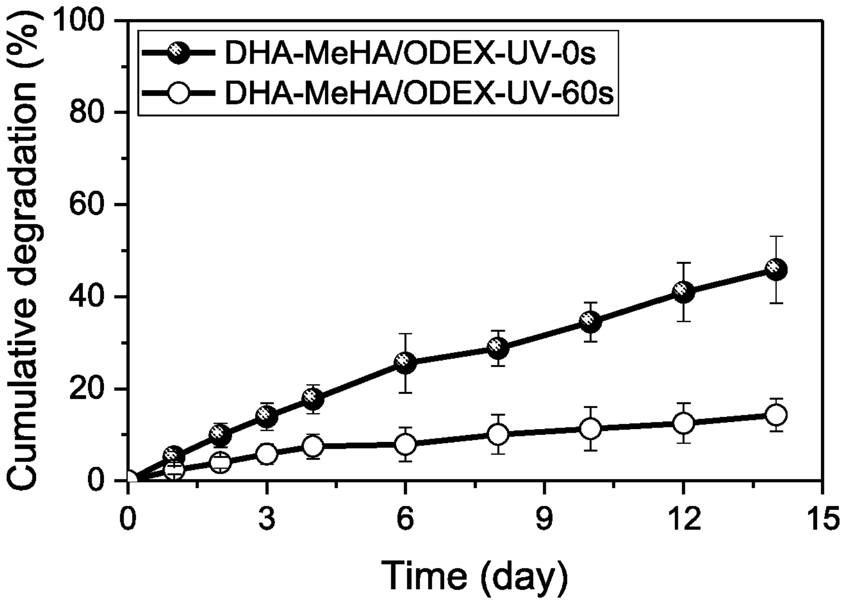


**Figure 4**

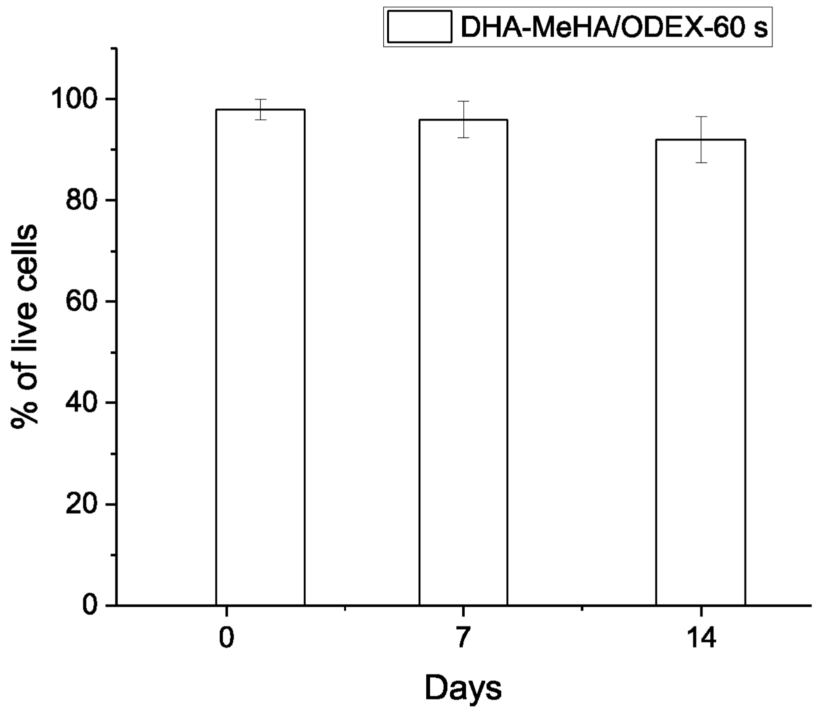


**Figure 5**

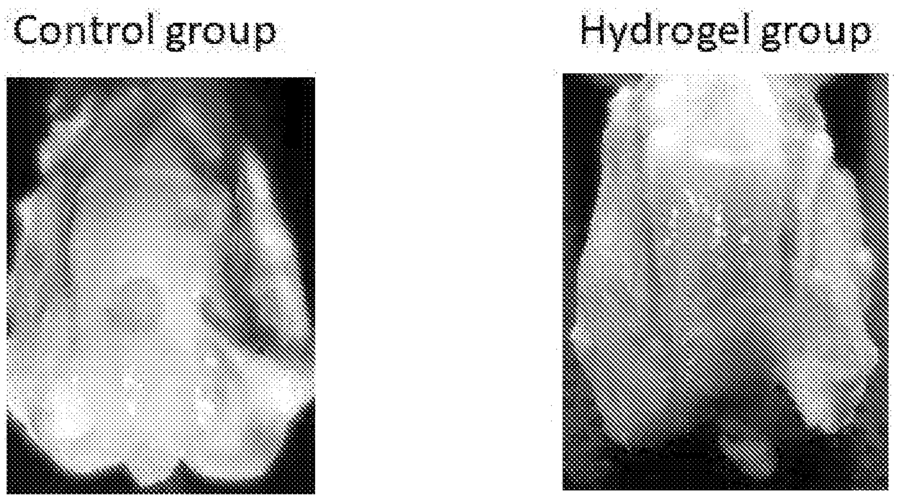


**Figure 6**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 104892962 A
- CN 106188584 A
- CN 104910396 A


### Non-patent literature cited in the description


- **YING LUO.** *Journal of Biomedical Materials Research B: Applied Biomaterials,* May 2010, vol. 93b (2), 387
- **HOANG D. LU.** *Adv Healthc Mater.,* July 2013, vol. 2 (7), 1028-36
- **SUDHIR KHETAN.** *Nat Mater.,* May 2013, vol. 12 (5), 458-65
- **AKON HIGUCHI et al.** *Chem. Rev.,* 2013, vol. 113, 3297-3328
- **AKON HIGUCHI et al.** *Chem. Rev.,* 2012, vol. 112, 4507-4540
- **BIJI BALAKRISHNAN et al.** *Chem. Rev.,* 2011, vol. 111, 4453-4474
- **C. SUN et al.** *Polymer,* 2019, vol. 160, 246-253
- **S.K. SEIDLITS et al.** *Biomaterials,* 2010, vol. 31, 3930-3940
- **PAUL BULPITT et al.** *Journal of biomedical materials research,* vol. 47 (2), 152-169
- **JIA X ; COLOMBO G ; PADERA R ; LANGER R ; KOHANE DS.** Prolongation of sciatic nerve blockade by in situ cross-linked hyaluronic acid. *Biomaterials,* 2004, vol. 25, 4797-4804
- **BOUHADIR KH ; HOUSMAN DS ; MOONEY DJP.** Synthesis of crosslinked poly(aldehyde guluronate) hydrogels. *Polymer,* 1999, vol. 40, 3575-3584